(19) Europäisches Patentamt
European Patent Office
Office européen des brevets

(11) **EP 1 323 405 B1**

(12) **EUROPEAN PATENT SPECIFICATION**

(45) Date of publication and mention
of the grant of the patent:
**08.08.2007 Bulletin 2007/32**

(21) Application number: **01963577.0**

(22) Date of filing: **10.09.2001**

(51) Int Cl.:
*A61K 6/00* (2006.01)　　*A61C 13/20* (2006.01)
*B22C 1/08* (2006.01)　　*B22C 9/04* (2006.01)
*B22C 1/00* (2006.01)

(86) International application number:
**PCT/JP2001/007866**

(87) International publication number:
**WO 2002/022081 (21.03.2002 Gazette 2002/12)**

(54) **DENTAL INVESTING MATERIAL AND METHOD OF PREPARING DENTAL MOLD**

DENTALDECKMATERIAL UND VERFAHREN ZUR HERSTELLUNG EINER DENTALFORM

MATERIAU POUR REVETEMENT DENTAIRE ET PROCEDE DE PREPARATION D'UN MOULE

(84) Designated Contracting States:
**BE CH DE LI**

(30) Priority: **14.09.2000 JP 2000280072**

(43) Date of publication of application:
**02.07.2003 Bulletin 2003/27**

(73) Proprietor: **NORITAKE CO., LIMITED**
**Nagoya-shi,**
**Aichi 451-8501 (JP)**

(72) Inventors:
• **KATO, Daisuke,**
**c/o NORITAKE CO., LTD.**
**Nagoya-shi, Aichi 451-8501 (JP)**
• **MATSUMOTO, Atsushi,**
**c/o NORITAKE CO., LTD.**
**Nagoya-shi, Aichi 451-8501 (JP)**

(74) Representative: **Kramer - Barske - Schmidtchen**
**European Patent Attorneys**
**Patenta**
**Radeckestrasse 43**
**81245 München (DE)**

(56) References cited:
**JP-A- 2 270 506**　　　　**JP-A- 5 246 818**
**JP-A- 63 039 806**　　　　**JP-B1- 35 003 298**

• **PATENT ABSTRACTS OF JAPAN vol. 0122, no. 52 (C-512), 15 July 1988 (1988-07-15) -& JP 63 039806 A (UBE IND LTD), 20 February 1988 (1988-02-20)**
• **DATABASE WPI Section Ch, Week 199344 Derwent Publications Ltd., London, GB; Class L02, AN 1993-349227 XP002307107 -& SU 1 770 021 A1 (ROSTNIITM RES ASSOC) 23 October 1992 (1992-10-23)**
• **PATENT ABSTRACTS OF JAPAN vol. 1999, no. 01, 29 January 1999 (1999-01-29) -& JP 10 262990 A (NORITAKE CO LTD), 6 October 1998 (1998-10-06)**

**Description**

TECHNICAL FIELD

The present invention relates to a dental investment material and a method for producing a dental investment mold having improved gas permeability.

BACKGROUND ART

In the dental treatment, a dental metal molded article such as a crown and inlay is employed. For producing such a metal molded article, a dental gypsum-bonded investment mold is employed. For producing such a dental gypsum-bonded investment mold, a suitable amount of water is added to a dental investment material, which is kneaded into slurry, which is then cast into a frame containing a wax pattern having a desired tooth pattern as a core and then allowed to solidify, and thereafter sintered at a high temperature.

A dental gypsum-bonded investment mold is advantageous since it can be readily cast into the frame, undergoes a less deformation due to the residual stress after sintering, allows a molded article to be released readily after casting and does not change significantly over time.

However, a dental gypsum-bonded investment mold allows a gas to be generated upon sintering at a high temperature due to the thermal decomposition of the gypsum or wax. Accordingly, a poor gas permeability of a dental investment mold allows a crack to be formed on the top of the mold after sintering at the high temperature. In addition, the air or other gases are trapped in the cavity by a molten metal pressed into the cavity and deprived of the room to escape, resulting in an increased pressure in the cavity, which leads to a stress focused on sharpened positions (such as margins) in the cavity, which results in a flash on the molded article.

In view of the problems described above, an objective of the invention is to provide a dental investment material and a method for producing a dental investment mold exhibiting an excellent gas permeability and capable of preventing the cracking of a mold article and the flash formation on a molded article. Document JP 63-39 806 A discloses an investment material comprising hemihydrate gypsum, a refractory material and calcium carbonate.

DISCLOSURE OF THE INVENTION

According to the one aspect of the invention, a dental investment material comprises a main component consisted of hemihydrate gypsum and a refractory material and 0.5 to 2.0% by weight of calcium carbonate mixed with the main component.

A dental investment material of the present invention contains a calcium carbonate. A calcium carbonate is decomposed to generate a carbon dioxide gas when exposed to a high temperature. A carbon dioxide gas forms a number of gas-permeable pores in a dental investment material, and is exhausted via such gas-permeable pores. Accordingly, a dental investment mold produced using a dental investment material of the present invention exhibits an excellent gas-permeability.

As a result, a dental investment mold produced using an inventive dental investment material of the present invention can prevent the cracking very reliably upon sintering. Thus, even when a shorter period for air-drying before sintering is provided prior to a rapid heating, an excellent dental investment mold capable of avoiding any damages such as cracks can be advantageously obtained. Such an advantage may be due to a higher gas permeability of a dental investment material of the present invention than that of a conventional dental investment material, which allows water vapor or other gases generated to be exhausted readily even when the investment material in a highly watery condition is heated rapidly, whereby avoiding any elevation of the pressure.

In addition, even when a metal material is cast under a high pressure into a resultant dental investment mold, a molded article having no damages such as a flash can be advantageously obtained at a high precision. Such an advantage may be due to the ability of tolerating a pressure higher than that employed conventionally since the gases generated upon casting is exhausted before establishing a high pressure as a result of a higher gas permeability of the dental investment mold.

According to the other aspect of the invention is a method for producing a dental investment mold comprising:

a step for installing, in a frame, a wax pattern consisting of a tooth pattern section having a desired tooth form and a support section which supports the tooth pattern section;
a step for casting a dental investment material into the frame while allowing one end of the support section to be intact without embedding but embedding the remainder of the wax pattern in the dental investment material; and,
a step for sintering the dental investment material to evaporate the wax pattern whereby forming a dental investment mold having a cavity which has the desired tooth form,

wherein a dental investment material comprises a main component consisted of hemihydrate gypsum and a refractory material and 0.5 to 2.0% by weight of calcium carbonate mixed with the main component.

BRIEF DESCRIPTION OF THE DRAWINGS

Fig. 1 shows a schematic view (a) to (f) illustrating a method for producing a dental investment mold according to Example 1.
Fig. 2 shows a schematic view illustrating a method for measuring the gas permeability of a dental investment mold of Example 2.

BEST MODE FOR CARRYING OUT THE INVENTION

In the first aspect of the present invention, the amount of the calcium carbonate in the dental investment material is 0.5 to 2.0% by weight. An amount less than 0.5% by weight may result in a reduction in the gas permeability of a dental investment mold, while an amount exceeding 2.0% by weight may result in the formation of a flash in a molded article although the reason is not clear.

Hemihydrate gypsum contained in the major component is obtained by sintering a gypsum dihydrate as a starting material under atmospheric pressure or under pressure to obtain a hemihydrate salt. Hemihydrate gypsum is cured when hydrated, by forming a structure in which the needle crystals of the gypsum dihydrate are tangled with each other, and serves as a binder of a dental investment mold. Hemihydrate gypsum can be classified into type α or type β.

A refractory material contained in the major component imparts a dental investment mold with an ability of tolerating a high temperature such as that upon casting a molten metal. A refractory material may be, for example, spinel, cristobalite, quartz, alumina, zirconia, magnesia and the- like.

It is preferable that, in the major component, hemihydrate gypsum is 80 to 20% by weight and a refractory material 20 to 80% by weight. When an amount of the hemihydrate gypsum exceeds 80% or an amount of the refractory material is less than 20%, there is a difficulty in obtaining an enough expansion to compensate for the casting-associated shrinkage of the metal to be casted. On the other hand, when an amount of the hemihydrate gypsum is less than 20% or an amount of the refractory material exceeds 80%, there is a possibility of occurrence of a crack which is caused by an excessive heating and expansion and an insufficient post-sintering strength.

More preferably, 40 to 25% of hemihydrate gypsum is combined with 60 to 75% of a refractory material.

It is preferable that 0.5 to 20 parts by weight of a gypsum whisker is further extra-added to a dental investment material described above. It serves to ensure a sufficient expansion whereby exerting a casting performance and a suitability sufficient for casting a metal having a high melting point.

An amount of a gypsum whisker added to a dental investment material which is less than 0.5 parts by weight or exceeds 20 parts by weight may lead to a difficulty in obtaining an appropriate inhibitory effect on shrinkage.

A gypsum whisker preferably has a diameter of 2 to 5 $\mu$m, a mean length of 10 to 100 $\mu$m and an aspect ratio of 10 to 50, for the purpose of exerting a further satisfactory expansion performance. An aspect ratio means a ratio of the length to the diameter of a gypsum whisker.

It is preferable to add a shrinkage-inhibiting additive, expanding agent, water reducing agent and setting time agent to a dental investment material, for the purpose of obtaining a precise dental investment mold in a desired form.

The second aspect of the invention is characterized by the use of a dental investment material containing a calcium carbonate to produce a dental investment mold.

A calcium carbonate is decomposed upon sintering to generate a carbon dioxide gas. As a result, a highly gas-permeable dental investment mold can be produced. In addition, the cracking of the dental investment mold can be also avoided.

A wax pattern has a tooth pattern section having a desired tooth form. The shape of the tooth pattern section can be formed for example by using an impression material to obtain a tooth form of a patient, using gypsum for modeling to obtain a pattern corresponding to the form of the impression material, melting and solidifying a wax on the pattern repetitively, and adjusting the form appropriately.

A support section provided in a wax pattern serves to support a tooth pattern section in the frame and because the wax pattern evaporates upon sintering after casting a dental investment material, the cavity having a shape identical to a desired tooth form is formed.

A wax pattern is embedded in a dental investment material, while one end of the support section of the wax pattern is exposed from the dental investment material, for the purpose of allowing the wax pattern to run out through this exposed part upon sintering the dental investment material.

A dental investment material is mixed with an appropriate amount of water and then cast into a frame. Upon allowing to stand, the dental investment material is solidified.

The calcination temperature needs a temperature at which a wax pattern can be vaporized and a calcium carbonate can be decomposed. Typically, the calcination temperature is preferably 750˚C to 850˚C. A temperature below 750˚C leads to a difficulty in decomposing a calcium carbonate, which may result in a dental investment mold whose gas permeability is poor. On the other hand, a temperature exceeding 850˚C may result in the decomposition of gypsum contained in a dental investment material.

Example 1

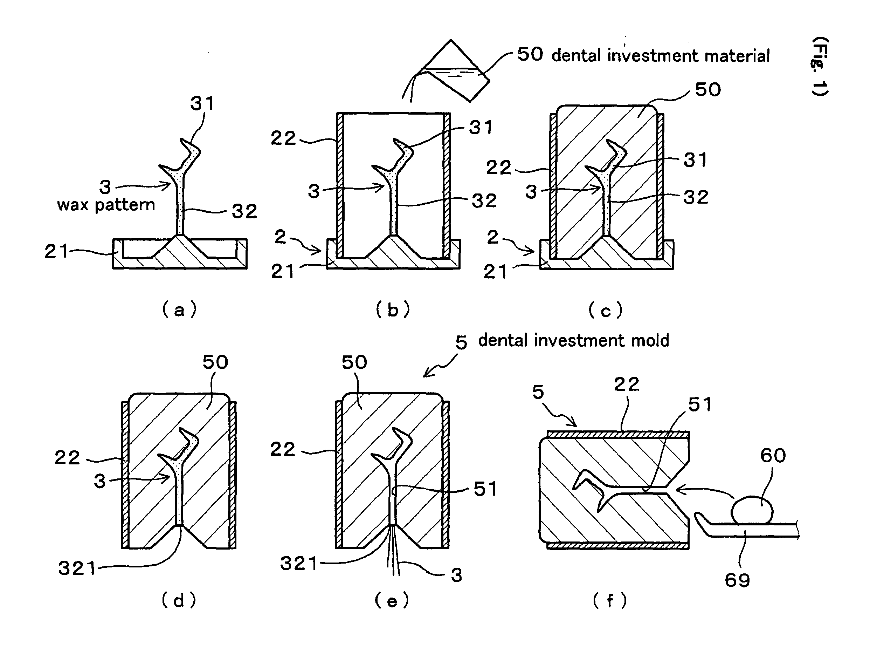

A dental investment material and a method for producing a dental investment mold according to the embodiment of the invention are discussed with referring to Fig. 1.

As shown in Table 1, the dental investment of this example comprises the major component consisting of 30.0 % by weight of a type $\alpha$ hemihydrate gypsum together with 65.0% by weight of spinel and 5.0% by weight of cristobalite as refractory materials. Based on 100 parts by weight of this major component, 1.0 part by weight of a calcium carbonate was contained together with 5.0 parts by weight of a gypsum whisker as an shrinkage-inhibiting additive, 1.6 parts by weight of ZrC as an expanding agent, 1.0 parts by weight of a water reducing agent as well as an appropriate amount of a retarding agent.

(Table 1)

| | name of components | | compounding ratio |
|---|---|---|---|
| major components | hemihydrate gypsum type $\alpha$ | | 30.0 |
| | refractory material | spinel | 65.0 |
| | | cristobalite | 5.0 |
| accessory components | gas permeability improver | calcium carbonate | 1.0 |
| | shrinkage-inhibiting additive | gypsum whisker | 5.0 |
| | expanding agent | ZrC | 1.6 |
| | water reducing agent | | 1.0 |
| | setting time agent | | appropriate |

A method for producing a dental investment mold using the dental investment material described above is discussed with referring to Fig. 1.

First, as shown in Fig. 1 (a), a tooth pattern section 31 having a form identical to the molded article to be obtained is molded by a standard method. A support section 32 integrated with the tooth pattern section 31 is also molded. As a result, a wax pattern 3 consisting of the tooth pattern section 31 and the support section 32 is obtained.

Then, as shown in Fig. 1 (b), the wax pattern 3 is installed in a standing manner on the center of a rubber lid 21, and a cylinder 22 is fixed on the rim of the lid 21. As a result, a frame 2 consisting of the lid 21 and the cylinder 22 is obtained.

Then, the dental investment material shown in Table 1 is combined with an appropriate amount of water to make a slurry. Subsequently, the dental investment material 50 in the form of a slurry is cast into the frame 2 whereby embedding the wax pattern 3.

As shown in Fig. 1 (c), the dental investment material 50 is cured by allowing to stand for about 30 minutes after casting the dental investment material.

As shown in Fig. 1 (d), the lid 21 is removed from the cured dental investment material 50. Upon this, the end 321 of the support section 32 of the wax pattern 3 is exposed from the dental investment material 50 since the support section 32 has been fixed on the lid 21.

Then the dental investment material 50 is sintered at 800˚C for 60 minutes. As a result, the wax pattern 3 is melted and runs out of the end 321 as shown in Fig. 1 (e), whereby forming a cavity 51 having a shape identical to the form of a molded article to be obtained.

As described above, a dental investment mold 5 is obtained.

As shown in Fig. 1 (f), a molten metal material 60 contained in a tray 69 is cast into the cavity 51 of the resultant dental investment mold 5 by a centrifugal casting method, whereby obtaining a molded article having a desirably shaped tooth form (crown). After cooling, the molded article is released.

The dental investment mold 5 produced using the dental investment material 50 of this example did not undergo any cracking upon sintering or casting. The resultant molded article had no flash.

Example 2

In this example, various dental investment materials were prepared with varying the amount of a calcium carbonate within the range from 0 to 2.0% by weight, and used to form dental investment molds, which were examined for the cracking, the flash in the resultant molded articles and the gas permeability. For comparison, a dental investment material containing 1.0% by weight of a magnesium carbonate instead of the calcium carbonate was examined similarly.

The method for preparing the dental investment materials and the method for producing the dental investment molds were similar to those in Example 1. Based on the quality and the condition of the resultant molded articles, the cracking of the dental investment molds and the flash formation of the molded articles were evaluated. The production of the molded articles was repeated 3 times for each dental investment material. Each dental investment material was designated by O when observing a satisfactory condition with no crack or flash, Δ when observing cracks and flashes which were problematic slightly but did not affect a practical use, and × when observing cracks and flashs which made a practical use impossible.

Subsequently, a sample 1 for measuring the gas permeability was produced using any of the dental investment material having the compositions described above, and subjected to the gas permeability test. The sample 1 for measuring the gas permeability was produced by adding 28% by weight of water to 100% by weight of a dental investment material having a composition described above, kneading to form a slurry, casting the slurry into a vinyl chloride pipe and allowing to solidify. Then the sample 1 was released 1 hour after casting, and sintered at 800˚C for 1 hour. The sample 1 was shaped as a cylinder whose outer diameter (a) was 50 mm and height (h) was 50 mm.

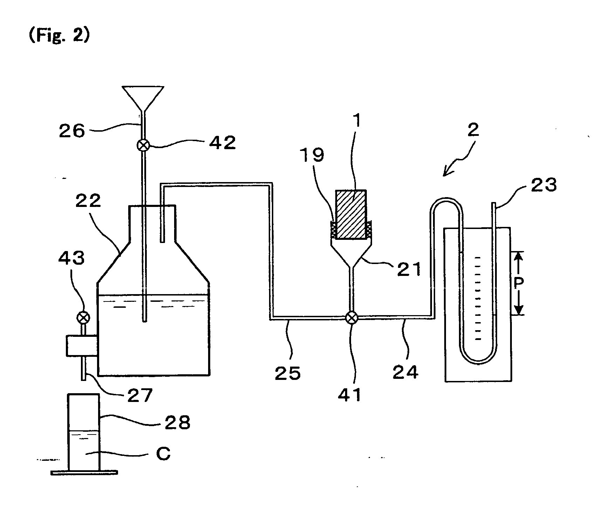

The outline of the gas permeability tester 2 shown in Fig. 2 is discussed below. The gas permeability tester 2 comprises of a sample mount 21 for setting the sample 1, a water tank 2 and a U-shaped tube 23. The bottom of the sample mount 21 was provided with a three-way valve 41, to which the water tank 22 and the U-shaped tube 23 are attached via connecting tubes 25 and 24, respectively. The water tank 22 was provided with a water supply tube 26 having a cock 42 and a water outlet 27 having a cock 43. The U-shaped tube 23 is used for a routine measurement of a differential pressure.

Then, for using this gas permeability tester 2 to measure the gas permeability actually, the side of the sample 1 is covered entirely with a wax, and the sample was placed in the sample mount 21 in the gas permeability tester 2, while filling the gap between the side of the sample 1 and the sample mount 21 with a wax 19 to ensure a tight contact. This wax 19 serves to block the gas permeation. As a result, the top and the bottom of the sample 1 were the only site enabling the gas permeation when being assembled as described above.

Then, the three-way valve 41 was turned to close the way to the sample 1 and communicate the water tank 22 with the U-shaped tube 23. At the same time, the cock 42 was closed and the cock 43 was opened. Then, any excessive water was allowed to run out from the water tank 22 to the pressure condition of the system was initialized. Thereafter, a graduated cylinder 28 was set beneath the water outlet 27 of the water tank 22.

Then, the three-way valve 41 was turned to open all of the three ways to communicate the sample 1, water tank 22 and U-shaped tube 23 with each other. Starting from this stage, the amount of water C ($cm^3$) released from the water outlet 27 within 1 minute was measured, with reading the differential pressure P (cm) from the U-shaped tube 23. Based on these values C and P, the gas permeability (cm/minutes) was calculated in accordance with the following equation.

$$\text{Gas permeability} = (C \times h)/(a \times P \times T)$$

wherein h is the height (cm) of the sample 1, a is the sectional area ($cm^2$) of the sample 1 and T is the testing time (min); thus h=5.0, a=2.5 $\times$ 2.5 $\times$ $\pi$ and T=1 in this test.

The results are shown in Table 2. As evident from Table 2, a higher amount of the calcium carbonate ($CaCO_3$) resulted in a higher gas permeability. On the other hand, the addition at 2.0% by weight resulted in a slight formation of flash.

(Table 2)

| amount of the carbonate (% by weight) | gas permeability (cm/minutes) | index* | crack | flash |
|---|---|---|---|---|
| 0% by weigh | 0.254 | 100 | Δ | × |
| $CaCO_3$ 0.5% by weight | 0.285 | 112 | ○ | ○ |

(continued)

| amount of the carbonate (% by weight) | gas permeability (cm/minutes) | index* | crack | flash |
|---|---|---|---|---|
| $CaCO_3$ 1.0% by weight | 0.310 | 122 | ○ | ○ |
| $CaCO_3$ 1.5% by weight | 0.321 | 127 | ○ | ○ |
| $CaCO_3$ 2.0% by weight | 0.336 | 132 | ○ | △ |
| $MgCO_3$ 1.0% by weight | 0.303 | 119 | △ | ○ |
| Index *; Index with 0% by weight being 100 | | | | |

Based on the results described above, a preferred amount of the calcium carbonate was proven to be 0.5 to 2.0 parts by weight, more preferably 0.5 to 1.5 parts by weight per 100 parts by weight of the main component consisting of the hemihydrate gypsum and the refractory material.

On the other hand, the addition of the magnesium carbonate resulted in an increased gas permeability of the dental investment mold but allowed the cracking to occur by some unknown reason.

INDUSTRIAL APPLICABILITY

As described above, the present invention can provide a dental investment material and a method for producing a dental investment mold, by which excellent gas permeability can be obtained and the cracking of a dental investment mold and the flash formation of a molded article can be prevented.

**Claims**

1. A dental investment material comprising:

   a main component consisted of hemihydrate gypsum and a refractory material; and **characterized in that** the investment material comprises 0.5 to 2.0% by weight of calcium carbonate mixed with the main component.

2. A dental investment material according to Claim 1,
   wherein 0.5 to 20 parts by weight of a gypsum whisker is further extra-added to the dental investment material.

3. A method for producing a dental investment mold comprising:

   a step for installing, in a frame, a wax pattern consisting of a tooth pattern section having a desired tooth form and a support section which supports the tooth pattern section;
   a step for casting a dental investment material into the frame while allowing one end of the support section to be intact without embedding but embedding the remainder of the wax pattern in the dental investment material; and,
   a step for sintering the dental investment material to evaporate the wax pattern whereby forming a dental investment mold having a cavity which has the desired tooth form,

   wherein a dental investment material comprises a main component consisted of hemihydrate gypsum and a refractory material and 0.5 to 2.0% by weight of calcium carbonate mixed with the main component.

**Patentansprüche**

1. Dentaleinbettmaterial, umfassend:

   eine Hauptkomponente, die aus Halbhydratgips und einem feuerfesten Material besteht, und **dadurch gekennzeichnet, dass** das Einbettmaterial 0,5 bis 2,0 Gew.-% Calciumcarbonat, das mit der Hauptkomponente gemischt ist, umfasst.

2. Dentaleinbettmaterial nach Anspruch 1, wobei dem Dentaleinbettmaterial ferner zusätzlich 0,5 bis 20 Gewichtsteile Gipsfaserkristalle zugesetzt sind.

3.  Verfahren zur Herstellung einer Dentaleinbettform, umfassend:

einen Schritt zum Installieren einer Wachsstruktur, die aus einem Zahnstrukturabschnitt mit einer gewünschten Zahnform und einem Trägerabschnitt, der den Zahnstrukturabschnitt stützt, besteht, in einem Rahmen,
einen Schritt zum Gießen eines Dentaleinbettmaterials in den Rahmen, während ein Ende des Trägerabschnitts ohne Einbetten intakt gelassen wird, jedoch der Rest der Wachsstruktur in dem Dentaleinbettmaterial eingebettet wird, und
einen Schritt zum Sintern des Dentaleinbettmaterials zum Verdampfen der Wachsstruktur, wodurch eine Dentaleinbettform mit einem Hohlraum, der die gewünschte Zahnform aufweist, gebildet wird,

wobei das Dentaleinbettmaterial eine Hauptkomponente, die aus Halbhydratgips und einem feuerfesten Material besteht, und 0,5 bis 2,0 Gew.-% Calciumcarbonat, das mit der Hauptkomponente gemischt ist, umfasst.

**Revendications**

1.  Matériau pour revêtement dentaire comprenant : un composant principal consistant en gypse hémihydraté et un matériau réfractaire, **caractérisé en ce que** le matériau de revêtement comprend de 0,5 à 2,0 % en poids de carbonate de calcium mélangé avec le composant principal.

2.  Matériau pour revêtement dentaire selon la revendication 1, dans lequel on ajoute en outre de 0,5 à 20 parties en poids de whiskers de gypse au matériau pour revêtement dentaire.

3.  Procédé pour produire un moule pour revêtement dentaire comprenant :

une étape pour installer, dans un cadre, un modèle en cire consistant en une section de modèle de dent ayant une forme de dent souhaitée et une section de support qui supporte la section de modèle de dent,
une étape pour mouler un matériau pour revêtement dentaire dans le cadre tout en permettant à une extrémité de la section de support d'être intacte sans enrobage mais en enrobant le reste du modèle en cire dans le matériau pour revêtement dentaire, et
une étape pour fritter le matériau pour revêtement dentaire afin d'évaporer le modèle en cire, formant ainsi un moule pour revêtement dentaire ayant une cavité qui a la forme de dent souhaitée,

dans lequel le matériau pour revêtement dentaire comprend un composant principal consistant en gypse hémihydraté et un matériau réfractaire et de 0,5 à 2,0 % en poids de carbonate de calcium mélangé avec le composant principal.

(Fig. 1)

50 dental investment material

5 dental investment mold

31
3
32
22
21
2
50
wax pattern

( a )

( b )

( c )

( d )

( e )

( f )

51
60
69

321

(Fig. 2)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 63039806 A